# NEUE EUROPÄISCHE PATENTSCHRIFT

(11) **EP 0 205 737 B2**
(45) Veröffentlichungstag und Bekanntmachung des Hinweises auf die Entscheidung über den Einspruch: **03.08.1994**
(45) Hinweis auf die Patenterteilung: 05.09.1990
(21) Anmeldenummer: 86101944.6
(22) Anmeldetag: 15.02.1986
(51) Int. Cl.: A61N 1/36, A61N 1/375, A61N 1/37

(54) **Herzschrittmacher**
Cardiac pacemaker
Entraîneur cardiaque

(30) Priorität: 19.06.1985 DE 3521874
(43) Veröffentlichungstag der Anmeldung: 30.12.1986
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Schmitt, Hans, Dipl.-Ing.

(56) Entgegenhaltungen:
- EP-A- 0 102 548
- EP-A- 0 119 596
- US-A- 3 198 195
- US-A- 3 683 932
- US-A- 3 871 382
- US-A- 4 479 489

## Beschreibung

Die Erfindung betrifft einen Herzschrittmacher gemäß dem Oberbegriff des Anspruchs 1. Ein derartiger Herzschrittmacher ist aus US-A-4479 489 bekannt. Hier dient der Zugang jedoch nur dem Zweck, die elektrische Leistung der Batterie des Herzschrittmachers zu überprüfen.

Derartige Herschrittmachersysteme sind seit langer Zeit in vielfältiger Form bekannt. Dennoch ergibt sich immer wieder, daß diese Systeme trotz sorgfältiger und vielgeübter Implantationstechnik ganz oder teilweise versagen. Es muß dann in für den Patienten unangenehmer Weise untersucht werden, worauf dieses Versagen zurückzuführen ist. Beispielsweise kann der Generator selbst versagen, wenn die Batterien vorzeitig erschöpft sind oder Schaltkreise ausfallen. Es treten aber auch Störungen dadurch auf, daß an der Elektrodenspitze z.B. im Herzen Reizschwellenerhöhungen durch Disloziierungen der Spitze oder daß Sensing-Probleme auftreten, so daß die Herzimpulse und die Impulse des Schrittmachergenerators nicht mehr synchron sind.

Besonders schwierig ist die Feststellung von derartigen Fehlern, wenn sie nur zeitweilig und mit unklaren Reaktionen beim Patienten auftreten. Bisher muß in solchen.

Fällen die Untersuchung des Generators und/oder der Elektrode über einen erneuten chirurgischen Eingriff mit allen damit zusammenhängenden Risiken erfolgen.

Der Erfindung liegt die Aufgabe zugrunde, einen Herzschrittmacher der eingangs erwahnten Art zu schaffen, der beim Auftreten von Schwierigkeiten oder Fehlern ohne operativen Eingriff untersucht und gegebenenfalls korrigiert werden kann.

Die überraschende Lösung dieser scheinbar unlösbaren Aufgabe wird durch die im kennzeichnenden Teil des Anspruchs 1 definierten Merkmale erreicht.

Treten Fehler in dem Herzschrittmachersystem auf, braucht der Patient zunächst keinem schwierigeren Eingriff ausgesetzt zu werden, als es beispielsweise dem Einstich einer Spritze entspricht. Es kann nämlich durch den erfindungsgemäßen Herzschrittmacher beispielsweise eine elektrische Leitung an den Impulsausgang des Generators geführt werden, um den Generator zum messen oder mit Zusatzimpulsen zu versehen. Diese elektrische Leitung kann dabei entweder über eine Kanüle oder unter Umstanden je nach ihrer Ausgestaltung auch direkt durch die Hautoberfläche und die Isolierung zu dem Zugang gebracht werden. Somit läßt sich zunächst einmal feststellen, ob der aufgetretene Fehler direkt am Generator oder aber an der Elektrode vorhanden ist.

Dabei ist es zweckmäßig, wenn die Isolierung selbstdichtend ist und vorzugsweise als Membran aus inertem Werkstoff, z.B. Silikon, ausgebildet ist. Nach Entfernen einer Kanüle od. dgl. ist somit diese Isolierung wieder dicht verschlossen.

Besonders zweckmäßig ist es, wenn die Isoliermembran zur Erleichterung der Lokalisierung und zu ihrer Aussteifung einen an ihrem Rand umlaufenden Wulst hat.

Eine Ausgestaltung der Erfindung von ganz erheblicher Bedeutung mit großen Vorteilen kann darin bestehen, daß die Elektrode einen inneren Kanal aufweist.

Dies ermöglicht es, uber den Zugang einen Mandrin in die Elektrode einzuführen, um diese eventuell nachträglich zu korrigieren und neu zu positionieren. Dabei kann ein solcher Mandrin ebenfalls über eine Kanüle zugeführt werden, die in den erwähnten Zugang eingestochen werden kann, ohne daß ein operativer Eingriff erforderlich ist.

Vor allem Bei Begrenzung eines solchen Hohlraumes am Ende der Elektrode ist es vorteilhaft, wenn die Dicke der Isolierung so groß ist, daß sie selbsttragend steif ist.

Eine besonders günstige Kombination ergibt sich, wenn die elektrisch leitende Fläche am Zugang ein Geflecht oder ein Netz ist.

Bei einer solchen Ausgestaltung ist sowohl das elektrische Nachmessen, das Erteilen zusätzlicher Impulse als auch der mechanische Zugang zu der Elektrode möglich. Ferner ist durch die Erfindung die Möglichkeit gegeben, mittels einer in den Zugang einstechbaren Kanüle auch ein Medikament zu applizieren. Dieses kann dann wesentlich gezielter und schneller beispielsweise über den Weg der implantierten Elektrode zum Herzen gelangen.

Eine konstruktiv besonders einfache Lösung ergibt sich, wenn die als leitende Fläche ausgebildete Wandung aus einer Metallplatte besteht.

Der erwähnte Hohlraum hat dann praktisch zwei Wandungen, deren eine elektrisch leitend ist, während die andere Wandung die durchstechbare Isolierung ist, die der Haut des Patienten näher liegt.

Ausgestaltungen der Erfindung insbesondere über die Anordnung des Hohlraumes mit durchstechbarer isolierender Wandung entweder mittelbar oder unmittelbar an dem Generator sind Gegenstand der Ansprüche 8 bis 11.

Vor allem bei Kombination einzelner oder mehrererdervorbeschriebenen Merkmale und Maßnahmen ergibt sich die Möglichkeit, den meist unmittelbar unter der Haut eines Patienten befindlichen Generator eines Herzschrittmachers durch einen einfachen Stich durch die Haut und die Isolierung zu erreichen und zu dem Zugang zu gelangen, so daß elektrische und/oder auch mechanische Maßnahmen möglich sind. Entweder kann auf diese Weise über den Zugang eine elektrische Leitung zum Nachmessen oder zum zusätzlichen Stimulieren eingeführt werden, oder es kann ein Mandrin zu der Elektrode gebracht oder auch ein Medikament zugeführt werden. Es könnte sogar eine Infusionsleitung für eine länger dauernde medikamentöse Leitung auf einfache Weise an diesem erfindungsgemäßen Zugang zu dem Herzschrittmachersystem installiert werden. Somit lassen sich zahlreiche unklare Erscheinungen bei Herzschrittmachern ohne operativen Eingriff abklären oder gegebenenfalls sogar korrigieren.

Nachstehend ist die Erfindung mit ihren ihr als wesentlich zugehörenden Einzelheiten anhand der Zeichnung in mehreren Ausführungsbeispielen noch näher beschrieben. Es zeigt in schematisierter Darstellung:
Fig. 1 eine Seitenansicht eines Herzschrittmachers mit einem Generator und einer daran über einen Elektrodenstecker angeschlossenen Elektrode, an deren herzfernem Ende ein Zugang unmittelbar angeordnet ist,
Fig. 2 eine Draufsicht der Anordnung nach Fig. 1 mit einem Querschnitt durch den Zugang zu dem Generator und dem Ende der Elektrode mit eingestochener Kanüle,
Fig. 3 eine etwa der Fig. 2 entsprechende Darstellung, wobei von dem Zugang eine Schlauchverbindung nach außen geführt und installiert ist,
Fig. 4 eine Anordnung mit einer fest installierten, in den Zugang eingestochenen Kanüle zum Anschließen einer Infusion,
Fig. 5 eine abgewandelte Ausführungsform eines Herschrittmachers mit zwei Elektroden, an deren Enden jeweils ein Zugang vorgesehen ist,
Fig. 6 eine abgewandelte Ausführungsform, bei welcher der Zugang seinerseits über einen Schlauch oder eine Elektrode mit dem Generator und der Elektrode verbunden ist, um beispielsweise näher zur Hautoberfläche als der Generator implantiert werden zu können,
Fig. 7 eine Ausführungsform etwa gemäß Fig. 6 mit zwei Elektroden, deren jede einen eigenen über einen Schlauch od. dgl. verbundenen Zugang hat, sowie
Fig. 8 eine Ausführungsform, bei welcher der Generator über eine Elektrode mit dem Zugang verbunden ist, von welchem die eigentliche Elektrode oder Zuleitung zum Herzen ausgeht.

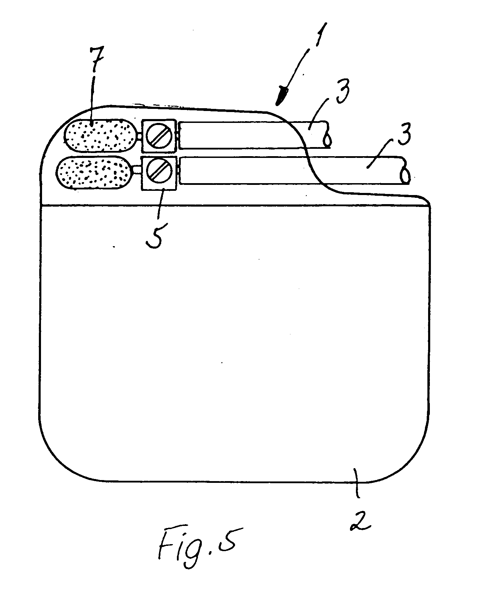

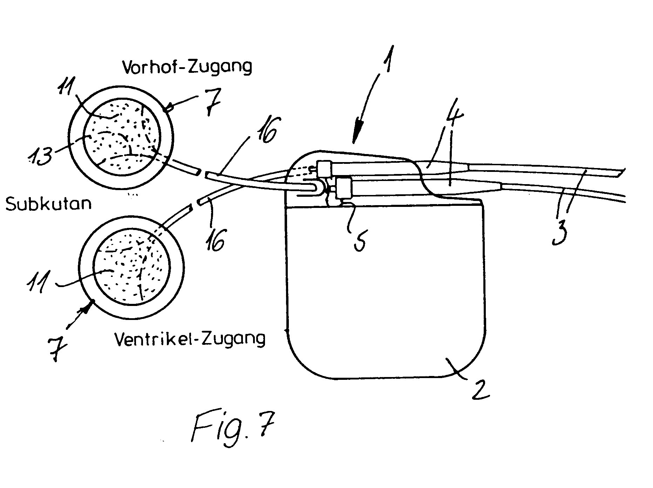

Ein im ganzen mit 1 bezeichneter Herzschrittmacher bzw. ein Herzschrittmachersystem weist in bekannter Weise einen subkutan implantierbaren Generator 2 und wenigstens eine in der Regel zum Herzen führende Elektrode oder Zuleitung 3 auf. Gemäß den Figuren 5 und 7 können auch mehrere Elektroden 3 von dem Generator 2 ausgehen.

Die Elektrode 3 ist über einen Stecker 4 am Impulsausgang 5 des Generators 2 angeschlossen. Erfindungsgemäß ist außerdem zu diesem Impulsausgang 5 des Generators 2 ein durch die Haut 6 des Patienten erreichbarer, im ganzen mit 7 bezeichneter Zugang vorgesehen, der eine mittels einer Kanüle 8 od. dgl. durchstechbare Isolierung 9 hat und von dieser abgeschlossen ist. Diese durchstechbare Isolierung 9 ist dabei in Gebrauchsstellung der Innenseite der Haut 6 des Patienten zugewandt. Somit kann beispielsweise über die Zuführkanüle 8 ein Medikament oder auch eine elektrische Leitung zugeführt werden. Dabei ist die Isolierung 9 selbstdichtend und vorzugsweise als Silikonmembran ausgebildet.

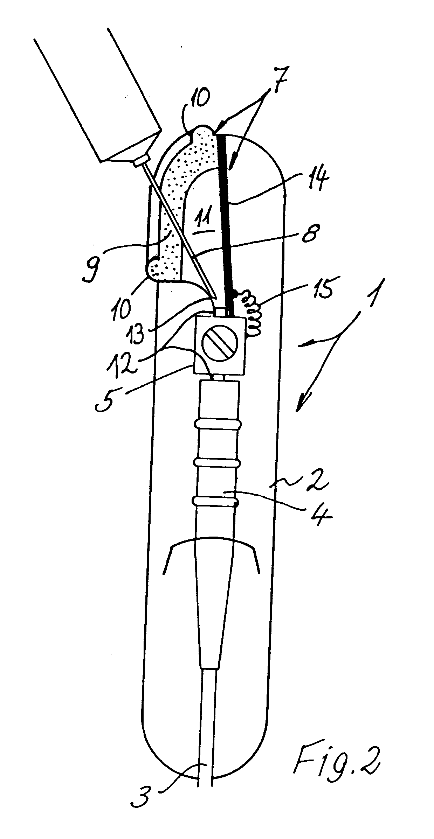

Vor allem in Fig. 2 erkannt man, daß diese Isoliermembran 9 zur Erleichterung der Lokalisierung und auch zu ihrer Aussteifung einen an ihrem Rand umlaufenden Wulst 10 hat.

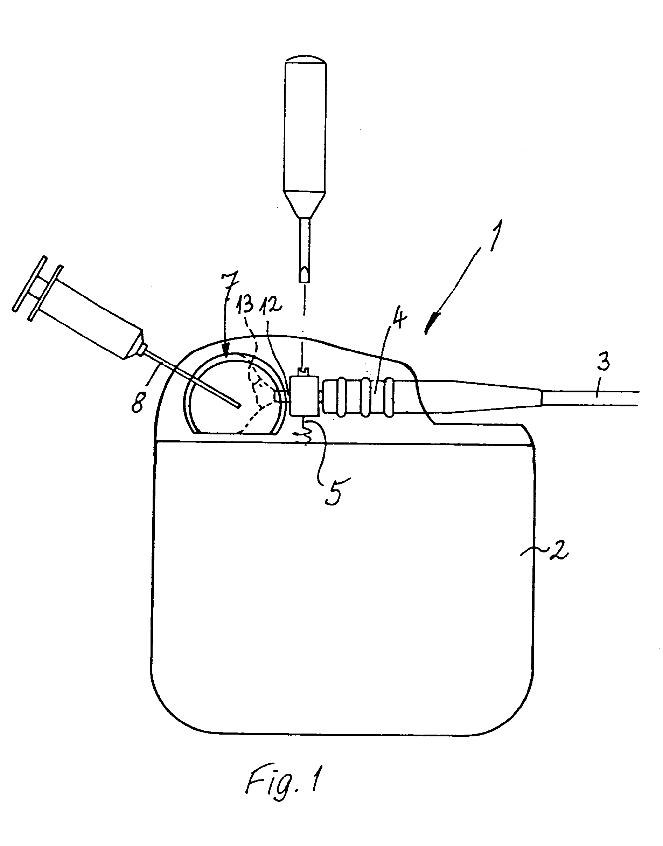

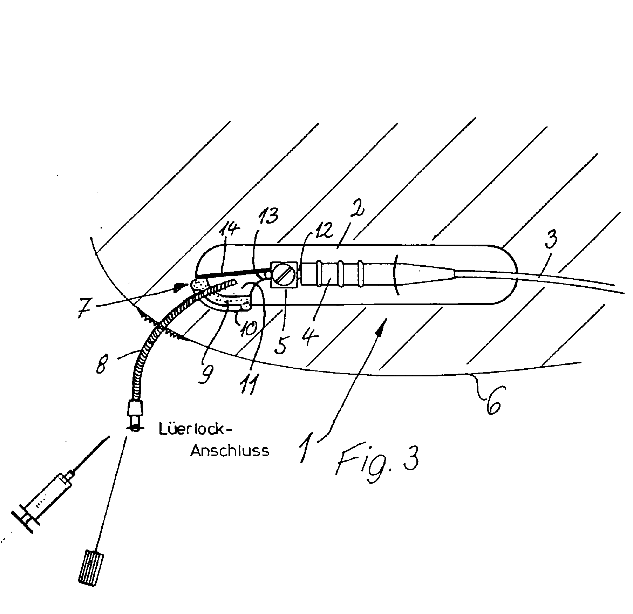

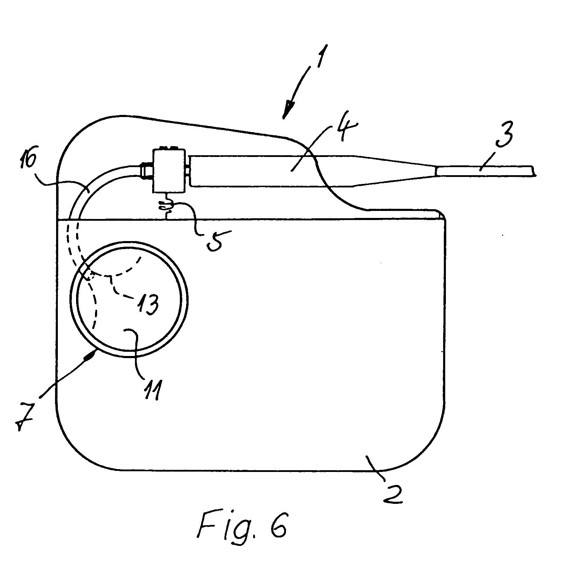

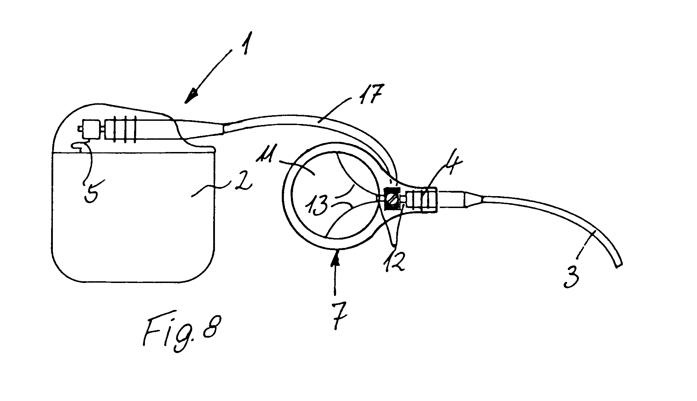

In allen dargestellten Ausführungsbeispielen begrenzt die Isolierung 9 einen Hohlraum 11 und dient gleichzeitig als ein Wandteil dieses Hohlraumes 11. Dieser Hohlraum 11 ist im Bereich des Elektrodenendes bzw. des Steckeranschlusses angeordnet und mittelbar oder unmittelbar mit dem Ende der Elektrode 3 verbunden, die in besonders zweckmäßigerWeise einen inneren Kanal für einen Mandrin aufweisen kann. Somit kann über die Kanüle 8 nicht nur ein Medikament oder eine elektrische Leitung, sondern auch ein derartiger Mandrin durch die Elektrode 3 bis zu deren Spitze geführt werden, um Korrekturen vorzunehmen. Gegebenenfalls kann sogar ein Mandrin ohne Kanüle unmittelbar durch die Haut 6 des Patienten in den Zugang 7 und von dort in die Elektrode 3 eingeführt werden. Dies ist vor allem dadurch erleichtert, daß der als Hohlraum 11 mit durchstechbarer isolierender Wandung 9 ausgebildete Zugang 7 in den dargestellten Ausführungsbeispielen in rückwärtiger Fortsetzung der Elektrode 3 an deren Stecker 4 angeschlossen ist und in seinem Inneren einen in das der Elektrode 3 abgewandte Steckerende 12 gerichteten Einführtrichter 13 hat. Man erkennt diese Gestaltung des Einführtrichters deutlich bei gemeinsamer Betrachtung der Figuren 1 und 2 oder 3, aber auch beispielsweise in Fig. 6 oder 8.

Fig. 2 veranschaulicht außerdem, daß die Dicke der Isolierung 9 so groß ist, daß sie nicht nur selbstdichtend, sondern auch selbsttragend steif ist und somit den Hohlraum 11 gegen ein Eindrükken sicher begrenzen kann.

Vor allem Fig. 2 verdeutlicht außerdem, daß der Zugang 7 zum Impulsausgang des Generators 2 einen elektrisch leitenden Bereich aufweist, der in irgendeiner Weise metallisch ist und dabei ein Geflecht oder ein Netz sein könnte, im Ausführungsbeispiel aber eine Metallplatte 14 ist, die mit der Elektrode 3 bzw. dem Impulsausgang direkt und/oder über eine elektrische Leitung 15 elektrisch leitend verbunden ist und in Gebrauchsstellung von der durchstechbaren Isolierung 9 abgedeckt ist. Es ergibt sich praktisch, daß eine Wandung des Hohlraumes 11 von der Isolierung 9 und die andere von der Metallplatte 14 gebildet ist, so daß eine die Isolierung 9 direkt oder über eine Kanüle 8 durchstoßende elektrische Leitung unmittelbar auf diese Metallplatte 14 trifft. Entsprechend leicht kann der Generator 2 gemessen oder mit Zusatzimpulsen versehen werden.

Neben der in den Figuren 1 bis 5 dargestellten unmittelbaren Anordnung des Zugangs 7 in rückwärtiger Fortsetzung der Elektrode 3 an dem Generator 2 ist es auch möglich, daß der vorzugsweise kammerförmige Hohlraum 11 mit Abstand zum Generator 2 implantierbar angeordnet ist und eine Verbindungsleitung 16 zu dem Elektrodenende bzw. dem Stecker 4 der Elektrode 3 hat. Man erkennt eine derartige Anordnung in Fig. 6 und für zwei Elektroden 3 in Fig. 7. Dies ermöglicht es, den, vorzugsweise als Hohlraum 11 ausgebildeten Zugang 7 möglichst günstig zu implantieren, wenn der Generator 2 aus bestimmten Gründen für die Zugänglichkeit des Zuganges 7 zu ungünstig zu liegen käme.

Vergleichbar ist in diesem Zusammenhang auch die Lösung nach Fig. 8. Dabei geht die z.B. zum Herzen führende Elektrode 3 von einer durchstechbaren Kammer 11 als Zugang 7 aus, die ihrerseits über eine Verbindungselektrode 17 mit dem Generator 2 verbunden ist.

Die Figuren 5 und 7 verdeutlichen, daß für jede zum Herzen führende Elektrode 3 ein eigener, vorzugsweise als Hohlraum 11 ausgebildeterZugang mit einer durchstechbaren Isolierung 9 vorgesehen sein kann.

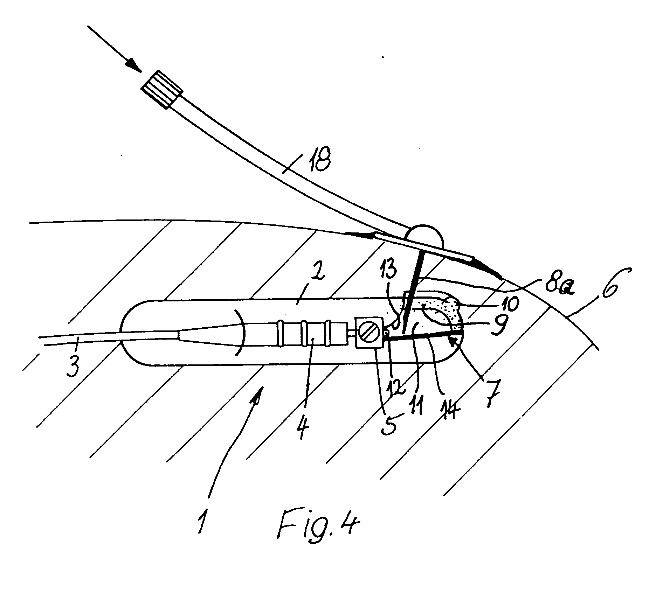

Neben der Möglichkeit, den Generator 2 oder die Elektrode 3 ohne operativen Eingriff zu untersuchen, indem mittelbar oder unmittelbar elektrische Leitungen, mechanische Hilfsmittel wie ein Mandrin od. dgl. durch die Haut zu dem Zugang 7 eingeführt werden können, ergibt sich gemäß beispielsweise Fig. 4 zusätzlich die Möglichkeit, über eine dauerhaft implantierte Kanüle 8 a und einen Schlauchanschluß 18 beispielsweise Medikamente durch eine Infusion zuzuführen. Somit erhält der Zugang 7 bei Ausbildung als Hohlraum 11 eine zusätzliche Funktion, weil derartige Medikamente dann wesentlich gezielter dem Weg der Elektrode 3 bzw. Zuleitung zum Herzen folgen kann und das Anlegen eines solchen Anschlusses von vorneherein erheblich erleichtert ist.

## Patentansprüche

1. Herzschrittmacher (1), bestehend aus einem i m-plantierbaren Generator (2) und wenigstens einer Elektrode (3) mit einer in Gebrauchssteilung der Innenseite der Haut des Patentienten zugewandten Isolierung (9), die durch die Haut durchstechbar ist, wobei ein von der Isolierung abgeschlossener Zugang (7) zum Impulsausgang (5) des Generators (2) für eine Zuführkanüle (8) und/oder für eine elektrische Leitung vorgesehen ist, und der Zugang (7) eine elektrisch leitende Fläche aufweist, die mit der Elektrode (3) bzw. dem Impulsausgang (5) elektrisch leitend verbunden ist und in Gebrauchsstellung von der durchstechbaren Isolierung (9) abgedeckt ist, dadurch gekennzeichnet, daß die Isolierung (9) einen Hohlraum (11) begrenzt und als Wandteil dieses Hohlraumes (11) dient, der im Bereich des Elektrodenendes angeordnet und mittelbar oder unmittelbar mit dem Ende der Elektrode (3),verbunden ist, daß wenigstens eine Wandung des von der durchstechbaren Isolierung (9) abgeschlössenen Hohlraumes (11) die elektrisch leitende Fläche bildet und daß diese Wandung mit der Elektrode (3) bzw. dem Impulsausgang (5) des Herzschrittmachers (1) elektrisch leitend verbunden ist.

2. Herzschrittmacher nach Anspruch 1, dadurch gekennzeichnet, daß die Isolierung (9) selbstdichtend ist und vorzugsweise als Membran aus inertem Werkstoff, z.B. Silikon, ausgebildet ist.

3. Herzschrittmacher nach Anspruch 1 oder 2, dadurch gekennzeichnet, daß die Isoliermembran (9) zur Erleichterung der Lokalisierung und zur Aussteifung einen an ihrem Rand umlaufende Wulst (10) hat.

4. Herzschrittmacher nach einem der Ansprüche 1 bis 3, dadurch gekennzeichnet, daß die Elektrode (3), einen inneren Kanal aufweist.

5. Herzschrittmacher nach einem der Ansprüche 1 bis 4, dadurch gekennzeichnet, daß die Dicke der Isolierung (9) so groß ist, daß sie selbsttragend steif ist.

6. Herzschrittmacher nach einem der Ansprüche 1 bis 5, dadurch gekennzeichnet, daß die elektrisch leitende Fläche am Zugang (7) eine metallische Fläche wie ein Geflecht oder ein Netz ist.

7. Herzschrittmacher nach einem der Ansprüche 1 bis 6, dadurch gekennzeichnet, daß die als leitende Fläche ausgebildete Wandung aus einer Metallplatte (14) besteht.

8. Herzschrittmacher nach einem der Ansprüche 1 bis 7, dadurch gekennzeichnet, daß dervorzugsweise als Hohlraum (11) mit durchstechbarer isolierender Wandung (9) und elektrisch leitender Wandung ausgebildete Zugang (7) in rückwärtiger Fortsetzung der Elektrode (3) an deren Stecker (4) angeschlossen ist und in seinem Inneren vorzugsweise einen in das der Elektrode (3) abgewandte Steckerende (12) gerichteten Einführtrichter (13) od. dgl. hat.

9. Herzschrittmacher nach einem der Ansprüche 1 bis 8, dadurch gekennzeichnet, daß dervorzugsweise kammerförmige Hohlraum (11) mit Abstand zum Generator (2) implantierbar angeordnet ist und eine Verbindungsleitung (16) od. dgl. zu dem Elektrodenende bzw. dem Stecker (4) der Elektrode (3) hat.

10. Herzschrittmacher nach einem der Ansprüche 1 bis 9, dadurch gekennzeichnet, daß die z.B. zum Herzen führende Elektrode (3) von einer durchstechbaren Kammer (11) od. dgl. ausgeht, die ihrerseits über eine Verbindungselektrode (17) mit dem Generator (2) verbunden ist.

11. Herzschrittmacher mit einem Generator und mehreren Herzelektroden nach einem der Ansprüche 1 bis 10, dadurch gekennzeichnet, daß für jede zum Herzen führende Elektrode (3) ein eigener, vorzugsweise als Hohlraum (11) ausgebildeter Zugang (7) mit einer durchstechbaren Isolierung (9) vorgesehen ist.

## Claims

1. A cardiac pacemaker (1) comprising an implantable generator (2) and at least one electrode (3) with an insulation (9) which in the position of use faces the inside of the patient's skin and is pierceable percutaneously, a means of access (7) to the pulse output (5) of the generator (2) and enclosed by the insulation being provided for a feed cannula (8) and/or an electric lead, and the means of access (7) has an electroconductive surface which is connected in an electroconductive manner to the electrode (3) and pulse output (5) and in the position of use is covered by the pierceable insulation (9), characterized in that the insulation (9) defines a cavity (11) and serves as a wall portion of said cavity (11), said cavity being disposed in the region of the electrode end and being directly or indirectly connected to the end of the electrode (3), that at least one wall of the cavity (11) enclosed by the pierceable insulation (9) composes the electroconductive surface, and that said wall is connected in an electroconductive manner to the electrode (3) and the pulse output (5) of the cardiac pacemaker (1).

2. Acardiac pacemaker as claimed in claim 1, characterized in that the insulation (9) is self-sealing and preferably takes the form of a membrane of inert material, e.g. silicone.

3. A cardiac pacemaker as claimed in claim 1 or claim 2, characterized in that the insulating membrane (9) has a bead (10) running round the edge thereof to facilitate localization and for stiffening.

4. A cardiac pacemaker as claimed in any one of claims 1 to 3, characterized in that the electrode (3) has an inner duct.

5. A cardiac pacemaker as claimed in any one of claims 1 to 4, characterized in that the insulation (9) is so thick as to be self-supportingly stiff.

6. A cardiac pacemaker as claimed in any one of claims 1 to 5, characterized in that the electroconductive surface at the means of access (7) is a metallic surface such as a mesh or a net.

7. A cardiac pacemaker as claimed in any one of claims 1 to 6, characterized in that the wall in the form of a conductive surface consists of a metal plate (14).

8. A cardiac pacemaker as claimed in any one of claims 1 to 7, characterized in that the means of access (7), preferably in the form of a cavity (11) with pierceable insulating wall (9) and electroconductive wall, is connected to the connector (4) of the electrode (3) in rearward extension of the latter and has in its interior preferably a lead-in funnel (13) or the like directed into the connector end (12) averted from the electrode (3).

9. A cardiac pacemaker as claimed in any one of claims 1 to 8, characterized in that the preferably chamber-like cavity (11) is arranged so as to be implantable in spaced relationship to the generator (2) and has a connecting lead (16) or the like to the electrode end and the connector (4) of the electrode (3).

10. A cardiac pacemaker as claimed in any one of claims 1 to 9, characterized in that the electrode (3) leading e.g. to the heart departs from a pierceable chamber(11) or the like, said chamber for its part being connected to the generator (2) via a connecting electrode (17).

11. A cardiac pacemaker including a generator and a plurality of heart electrodes as claimed in any one of claims 1 to 10, characterized in that a separate means of access (7) preferably in the form of a cavity (11) and having a pierceable insulation (9) is provided for each electrode (3) leading to the heart.

## Revendications

1. Stimulateur cardiaque (1), constitué d'un générateur implantable (2) et d'au moins une électrode (3) avec une isolation (9) qui, en position d'utilisation, est tournée vers la peau du patient, et qui peut être transpercée à travers la peau, un accès (7) à la sortie d'impulsions (5) du générateur (2), accès qui est fermé par l'isolation (9), étant prévu pour une canule d'alimentation (8) et/ou pour une ligne électrique, et cet accès (7) présentant une surface électroconductrice, qui est reliée électriquement à l'électrode (3) ou encore à la sortie d'impulsions (5), et qui, en position d'utilisation, est recouverte par l'isolation transperçable (9), caractérisé en ce que l'isolation (9) délimite une cavité (11) et sert d'élément de paroi de cette cavité (11), laquelle est disposée dans la région de l'extrémité de l'électrode et est reliée directement ou indirectement à l'extrémité de l'électrode (3), en ce qu'au moins une paroi de la cavité fermée par l'isolation (9) transperçable forme la surface électroconductrice, et en ce que cette paroi est reliée de manière électroconductrice à l'électrode (3) ou à la sortie d'impulsions (5) du stimulateur cardiaque (1).

2. Stimulateur cardiaque selon la revendication 1, caractérisé en ce que l'isolation (9) est auto- étanchéifiante, et est réalisée sous la forme d'une membrane en matériau inerte, par exemple en silicone.

3. Stimulateur cardiaque selon la revendication 1 ou 2, caractérisé en ce que la membrane isolante (9) possède un bourrelet (10) entourant son bord, qui est destiné à faciliter sa localisation et à la renforcer.

4. Stimulateur cardiaque selon l'une des revendications 1 à 3, caractérisé en ce que l'électrode (3) présente un canal intérieur.

5. Stimulateur cardiaque selon l'une des revendications 1 à 4, caractérisé en ce que l'épaisseur de l'isolation (9) est suffisante pour qu'elle soit rigide et autoportante.

6. Stimulateur cardiaque selon l'une des revendications 1 à 5, caractérisé en ce que la surface électroconductrice prévue sur l'accès (7) est une surface métallique telle qu'une grille ou un treillis.

7. Stimulateur cardiaque selon l'une des revendications 1 à 6, caractérisé en ce que la paroi réalisée sous la forme de surface électroconductrice est formée par une plaque métallique (14).

8. Stimulateur cardiaque selon l'une des revendications 1 à 7, caractérisé en ce que l'accès (7), réalisé de préférence sous la forme d'une cavité (11) avec une paroi isolante transperçable (9) et une paroi électroconductrice, est raccordé, dans le prolongement de l'électrode (3) vers l'arrière, au connecteur (4) de cette dernière, et possède intérieurement un cône d'introduction (13) ou analogue dirigé dans l'extrémité (12) du connecteur qui est éloignée de l'électrode (3).

9. Sti mulateur cardiaque selon l'une des revendications 1 à 8, caractérisé en ce que la cavité (11) de préférence en forme de chambre est conçue implantable à distance du générateur (2), et possède une ligne de jonction (16) ou analogue menant à l'extrémité de l'électrode ou encore au connecteur (4) de l'électrode (3).

10. Sti mulateur cardiaque selon l'une des revendications 1 à 9, caractérisé en ce que l'électrode (3), qui mène par exemple au coeur, part d'une chambre transperçable (11) ou analogue, qui est elle-même reliée au générateur (2) par l'intermédiaire d'une électrode de liaison (17).

11. Sti mulateur cardiaque avec un générateur et plusieurs électrodes cardiaques selon l'une des revendications 1 à 10, caractérisé en ce qu'il est prévu, pour chaque électrode (3) menant au coeur, un accès (7) individuel réalisé de préférence sous la forme d'une cavité (11) et muni d'une isolation transperçable (9).
